# EUROPEAN PATENT APPLICATION

(11) **EP 2 047 760 A1**
(43) Date of publication of application: **15.04.2009**
(21) Application number: 07791356.4
(22) Date of filing: 26.07.2007
(51) Int. Cl.: A23L 1/30, A23K 1/16, A61K 31/045, A61K 31/715, A61K 36/75, A61P 39/06, C12P 23/00

(54) **ORAL COMPOSITION ENABLING INCREASED ABSORPTION OF CRYPTOXANTHIN**

(30) Priority: 28.07.2006 JP 2006206315
(71) Applicant: UNITIKA LTD., Amagasaki-shi, Hyogo 660-0824 (JP)
(72) Inventor: TAKAYANAGI, Katsuhikoc/oUNITKA LIMITED, Kyoto 611-021 (JP); MUKAI, Katsuyukic/o UNITKA LIMITED, Kyoto 611-0021 (JP)
(74) Representative: HOFFMANN EITLE
(86) International application number: PCT/JP2007/064656
(87) International publication number: WO 2008/013219

(57) **Abstract**

A composition having improved absorbability, into the living body, of cryptoxanthin derived from a natural product, particularly that derived from *Citrus unshiu* known to be rich in cryptoxanthin, and a method for economically and conveniently producing the same are provided. As a cryptoxanthin highly absorbable oral administration composition, which is a composition comprising natural product-derived cryptoxanthin, wherein the amount of dietary fibers contained in said composition is 400 times by weight or less based on cryptoxanthin, this composition is a composition wherein the absorbability of cryptoxanthin into the living body is improved 2 times or more in comparison with the case of directly ingesting a natural product containing said cryptoxanthin.

## Description

### TECHNICAL FIELD

The present invention relates to a highly absorbable oral administration composition with improved absorbability of cryptoxanthin into the living body, which can be used in a food, a feed, a pharmaceutical preparation, and the like.

### BACKGROUND ART

Cryptoxanthin is a carotenoid specifically contained in *Citrus unshiu* and persimmons and is also the major carotenoid found in blood. Though usefulness of cryptoxanthin is clear from animal tests and epidemiological investigations, its recognition is low and also its commercialization is not advancing because of its insufficient supply, low absorbability into the living body and the like, which are different from the carotenoids such as β-carotene, lycopene and the like.

Since the absorption rate of fat-soluble nutrient substances such as a carotenoid and the like is generally low, various efforts have been made for improving the absorbability. For example, it has been known for a long time as a cooking method that absorption efficiency of the carotenoid becomes high in the pan fried or deep fried case using oil than the case of eating as raw or after boiling.

Also, as a method for improving absorbability in the case of ingesting a carotenoid not as a food but as a supplement or the like, there is a report stating that an emulsified product which satisfy a certain condition is effective (e.g., see Patent Reference 1). However, since this method requires extraction of a raw material containing a carotenoid with an organic solvent or the like in advance, not only the process becomes complex and the cost becomes high, but also it has an aspect of lowering the yield.

In addition, there is a method for improving digestion absorption efficiency from the intestinal tract, by ingesting not only a carotenoid but also a combination of probiotics or the like and an antioxidant such as a carotenoid or the like (e.g., see Patent Reference 2). However, since this method uses the carotenoid as an anti-oxidation component, it did not lead to the improvement of the absorption rate of the carotenoid.

As described in the above, methods for improving absorption efficiency are known on some carotenoids, but it was hard to say that they are sufficient for conveniently improving absorbability and/or absorption rate of a rare carotenoid such as β-cryptoxanthin or the like into the living body.
On the other hand, the present inventors have filed patent applications on various uses, extraction methods and the like about cryptoxanthin. That is, they are a feed for poultry raising use in which a substance having high content of cryptoxanthin derived from a fruit of citrus fruits is formulated in the feed (cf. Patent Reference 3), an oral administration composition having a whitening effect useful in preventing and improving pigmentation, liver spots, freckles and the like of the skin, which contains cryptoxanthin and cysteine (cf. Patent Reference 4), a skin external preparation having a melanin formation inhibitory effect, which contains cryptoxanthin as an active ingredient (cf. Patent Reference 5), an anti-osteoporosis composition which contains cryptoxanthin and a flavonoid (cf. Patent Reference 6), and a method for extracting cryptoxanthin by adding an organic solvent after applying an enzyme treatment by adding the enzyme (cf. Patent Reference 7).
However, none of the above-mentioned cases describe the improvement of absorbability into the living body by lowering the weight ratio of dietary fibers to cryptoxanthin to a certain value or less.
Patent Reference 1: JP-A-9-157159
Patent Reference 2: JP-A-2005-34135
Patent Reference 3: JP-A-2003-52338
Patent Reference 4: JP-A-2006-104088
Patent Reference 5: JP-A-2006-104089
Patent Reference 6: JP-A-2006-104090
Patent Reference 7: JP-A-2005-27520

### DISCLOSURE OF THE INVENTION

### PROBLEMS THAT THE INVENTION IS TO SOLVE

The present invention aims at providing a composition having improved absorbability into the living body, of cryptoxanthin derived from a natural product, particularly that derived from *Citrus unshiu* known to be rich in cryptoxanthin, and a method of producing the same economically and conveniently.

### MEANS FOR SOLVING THE PROBLEMS

The present inventors have conducted extensive studies with the aim of solving these problems and, as a result, found that the problems can be solved by lowering the weight ratio of dietary fibers to cryptoxanthin to a certain value or less.

That is, the first gist of the present invention resides in a highly absorbable oral administration composition of cryptoxanthin, which is a composition comprising natural product-derived cryptoxanthin, wherein the amount of dietary fibers contained in said composition is 400 times by weight or less based on cryptoxanthin; preferably that wherein absorptiveness of cryptoxanthin into the living body is improved 2 times or more in comparison with the case of directly ingesting a natural product containing said cryptoxanthin; and also preferably that wherein the natural product is *Citrus unshiu*; which is also preferably the aforementioned highly absorbable oral administration composition of cryptoxanthin, wherein a part or whole of cryptoxanthin is dissolved in an oil or fat; also preferably the aforementioned highly absorbable oral administration composition of cryptoxanthin, wherein a part or whole of cryptoxanthin is emulsified by an emulsifying agent.

The second gist of the present invention resides in a method of producing the aforementioned highly absorbable oral administration composition of cryptoxanthin, which comprises subjecting a natural product or a processed product thereof to an enzyme treatment by adding the enzyme, subjecting the resulting enzyme-treated product to a solid-liquid separation to obtain the residue, and obtaining an oral administration composition from said residue.

The third gist of the present invention resides in a method of producing the aforementioned highly absorptive oral administration composition of cryptoxanthin, which comprises extracting cryptoxanthin by adding an organic solvent to the natural product or the processed product thereof, and an oral administration composition is obtained from said extract.

The fourth gist of the present invention resides in a food or drink or a feed, which comprises the aforementioned highly absorbable oral administration composition of cryptoxanthin. In addition, the fifth gist of the present invention resides in a pharmaceutical preparation, which comprises the aforementioned highly absorptive oral administration composition of cryptoxanthin as an active ingredient.

### EFFECT OF THE INVENTION

According to the present invention, cryptoxanthin can be absorbed into the living body efficiently with a small amount of ingestion, so that it is not necessary to ingest a large amount of a natural product which contains cryptoxanthin, and what is more, it can be safely ingested. In addition, because of this, an effect that formulation design become easy when formulated in foods and drinks may be cited, and as a result, effects of easily increasing serum cryptoxanthin, improving anti-oxidation ability of the living body and the like can be obtained.

### BRIEF DESCRIPTION OF THE DRAWINGS

Fig. 1 is a graph showing comparison of the serum β-cryptoxanthin levels in the ingestion groups of hard capsules respectively produced from the composition of Example 1 and the composition of Comparative Example 1 (Test Example 1).
Fig. 2 is a graph showing the absorption amount ratio of serum β-cryptoxanthin in the ingestion group of hard capsules produced from the composition of Example 1, when the increased amount of the serum β-cryptoxanthin level in the ingestion group of hard capsules produced from the composition of Comparative Example 1 was regarded as 1 (Test Example 1).
Fig. 3 is a graph showing comparison of the serum β-cryptoxanthin levels in the ingestion groups of hard capsules respectively produced from the composition of Example 1 and the composition of Comparative Example 1 (Test Example 2).
Fig. 4 is a graph showing the absorption amount ratio of serum β-cryptoxanthin in the ingestion group of hard capsules produced from the composition of Example 1, when the increased amount of the serum β-cryptoxanthin level in the ingestion group of hard capsules produced from the composition of Comparative Example 1 was regarded as 1 (Test Example 2).
Fig. 5 is a graph showing comparison of the serum β-cryptoxanthin levels after ingestion in the ingestion groups of hard capsules and soft capsules respectively produced from the composition of Example 1 and the composition of Comparative Example 1.
Fig. 6 is a graph showing comparison of the serum β-cryptoxanthin levels in the ingestion group of a soft drink produced from the emulsifying agent in Example 2 and the ingestion group of *Citrus unshiu* juice of Comparative Example 2 (Test Example 3).
Fig. 7 is a graph showing the absorption amount ratio of serum β-cryptoxanthin in the ingestion group of the soft drink produced from the emulsifying agent in Example 2, when the increased amount of the serum β-cryptoxanthin level in the ingestion group of the *Citrus unshiu* juice of Comparative Example 2 was regarded as 1 (Test Example 3).

### BEST MODE FOR CARRYING OUT THE INVENTION

The following describes the present invention in detail.

According to this description, all of the ratios, percentages and the like are by weight, unless otherwise noted.
There are α type and β type structural isomers with respect to cryptoxanthin. In addition, cryptoxanthin exists in an ester form with a fatty acid other than in the free form, and its molecular species vary depending on the chain length of the fatty acid bonded. The cryptoxanthin according to the present invention is not particularly limited and may be either the above-mentioned cryptoxanthin or fatty acid ester thereof. Among them, β-cryptoxanthin and a fatty acid ester thereof are preferable because they are contained in large amounts in oranges, persimmons and the like.

As the supply source of cryptoxanthin according to the present invention, natural vegetables, fruits and the like are preferable. Among them, *Citrus unshiu* is preferable as the supply source, because the β-cryptoxanthin content is high in comparison with other vegetables and fruits and the production is also high.

The first cryptoxanthin highly absorbable oral administration composition of the present invention is a composition which contains the above-mentioned natural product-derived cryptoxanthin wherein the amount of dietary fibers contained in said composition is in a certain amount or less.

The dietary fibers are generally defined as "totality in food, which is not degraded by human digestive enzymes", and plant-derived insoluble dietary fibers such as cellulose, hemicellulose, pectin, lignin and the like, insoluble dietary fibers derived from crustaceans and mushrooms such as chitin and the like, plant-derived water-soluble dietary fibers such as guar gum, mannan and the like, seaweed-derived water-soluble dietary fibers (seaweed polysaccharides) such as alginic acid, fucoidin and the like, collagen, chondroitin sulfate and the like derived from an animal connective tissue may be cited.

The amount of dietary fibers according to the present invention means a value measured by the Prosky method. The Prosky method is a method in which a defatted food is treated with a carbohydrate-degrading enzyme and a protease, and an amount of the residual organic components is determined, and its details are described in AOAC (Association of Official Analytical Chemists) [Y.L., et al., J. Assoc. Off. Anal. Chem., 67, 1044 - 1051 (1984)].

According to the present invention, it is necessary that the amount of the dietary fibers contained in said composition should be 400 times or less by weight of cryptoxanthin. It is preferably 200 times or less, more preferably 100 times or less. Amounts exceeding 400 times cannot be employed, because the absorbability of cryptoxanthin by the living body becomes poor and the effect of the present invention is not exerted. In this connection, a high-performance liquid chromatography (HPLC) was used in the measurement of the cryptoxanthin quantity. That is, this was carried out by using LC-10A manufactured by Shimadzu Corp. as the HPLC device, connecting a Resolve C18 (φ 3.9 x 150 mm) column manufactured by Waters, introducing a sample to which the same amount of methanol was added and using methanol:ethyl acetate = 7:3 as the mobile phase, and at a column temperature of 30°C, a flow rate of 1.0 ml/min and the detection wavelength of 450 nm.

Also, according to the cryptoxanthin highly absorbable oral administration composition of the present invention, the absorbability of cryptoxanthin into the living body is improved 2 times or more in comparison with the case of ingesting the natural product as such in which cryptoxanthin is contained. In this connection, the "absorbability" as used in the present invention means a result of allowing healthy persons to ingest the composition of the present invention or a cryptoxanthin-containing comparative food (persimmon, *Citrus unshiu* or the like) continuously every day for 4 weeks and comparing the serum cryptoxanthin quantities measured before the ingestion and 4 weeks thereafter. The serum cryptoxanthin quantity is a value measured by the above-mentioned HPLC method by separating sera from the collected whole blood.

In addition, "to ingest a natural product as such" means that its ingestion after operations such as squeezing, drying, pulverization and the like is also included, which means that it is ingested under a state of not applying an enzyme treatment or an extraction operation with an organic solvent.

Next, production methods of the cryptoxanthin highly absorbable oral administration composition are described.

As the first method, there is a method in which an enzyme reaction is applied to the aforementioned natural product or a processed product thereof by adding an enzyme. As the processed product of a natural product, a residue after squeezing and the like may be cited. Such a residue is prepared, for example, by squeezing a fruit with an inline squeezer, chopper helper squeezer, Brown squeezer or the like, preparing a fruit juice therefrom by filtration or screening using a paddle type or screw type finisher or the like or by centrifugation, and then collecting the resulting solid.

According to the present invention, the enzyme to be used in the enzyme treatment is not particularly limited as long as it can degrade the organic matters contained in the squeezed residue, particularly biopolymers and the like which constitute cell walls and the like and which can be dietary fibers, and amylase, glucoamylase, cellulase, hemicellulase, pectinase, mannanase, xylase, protease, peptidase, lipase, mallation enzyme (cell wall disintegrating enzyme) and the like are used. Among them, carbohydrate-degrading enzymes such as cellulase, hemicellulase, pectinase, mannanase, xylanase and mallation enzyme are desirable because of their high efficiencies to reduce the quantity of dietary fibers.

As the enzymes to be added, purified enzymes thereof may be used, or bacterial cells or cultures of microorganisms showing the activities or crudely purified products thereof may be used. These enzymes may be used alone, or two or more enzymes may be used by mixing them. Amount of the enzyme to be added is not particularly limited and it may be added depending on the reactivity of the enzyme. For example, in the case that pectinase is used, it is preferably from 1 to 100,000 units, more preferably from 10 to 10,000 units, based on 100 g of the material to be treated.

After adding the above-mentioned enzyme, the enzyme reaction is advanced by uniformly mixing the enzyme and the object matter by stirring or the like. Regarding the reaction temperature in this case, it is preferable to carry out the reaction under such a condition that the enzyme is not inactivated and rotting hardly occurs and also under such a condition that cryptoxanthin is not lost. Illustratively, the temperature is from 0 to 90°C, preferably from 0 to 80°C, more preferably from 0 to 70°C. Regarding pH for the reaction, it is preferable to carry out the reaction under the optimum condition of the enzyme, as a matter of course, and pH from 2 to 12, preferably pH from 2.5 to 8, is suitable. The reaction time depends on the amount of the material to be treated and the enzyme, and it is preferable from the operational point of view to set the time to generally from 1 to 48 hours. In carrying out the reaction, the reaction may be carried out with stirring the material to be treated, or it may be a static reaction.

After completion of the enzyme treatment, the enzyme-treated material may be used as such, or it may be further subjected to a certain processing and then used. Illustratively, a residue after solid-liquid separation of the enzyme-treated material, a dried product of the residue after solid-liquid separation, a dried product of the reaction material as such without solid-liquid separation may be used. In addition, an extract of components and the like using a solvent, water, super critical carbon dioxide or the like may also be used. Further, the impurities may subsequently be removed. As the method for removing impurities, for example, washing with water, washing with an organic solvent, a method for passing through a silica gel column, resin column, reverse phase column or the like, treatment with activated carbon, partition by solvents having different polarities, recrystallization, vacuum distillation and the like may be cited. Particularly, a water washing in which the enzyme-treated material is subjected to solid-liquid separation, and the solid matter is stirred after water is added again and then subjected to solid-liquid separation is a preferable method, because reaction products such as sugar and the like produced by the enzyme treatment can be easily removed.

As the second method of the production method of the cryptoxanthin highly absorbable oral administration composition, a method in which cryptoxanthin is extracted by adding an organic solvent to a natural product or a processed product thereof may be cited. As the processed product of the natural product, a residue after squeezing of *Citrus unshiu* fruits and/or an enzyme-treated product obtained by applying the above-mentioned enzyme treatment to the squeezed residue may be cited.

The organic solvent used in the extraction is not particularly limited as long as it can be used in producing food. In addition to the hexane and acetone for oil and fat extraction, ethanol allowed as a food additive and the like, edible oils and fats such as safflower oil, olive oil, rapeseed oil, soybean oil, corn oil, coconut oil, sunflower oil, rice oil, fish oil, beef tallow, lard, MCT and the like may also be used. Among these, use of ethanol which has high ability to dissolve cryptoxanthin and is easy to handle is particularly preferable.

Regarding the ratio of the material to be subjected to extraction such as a natural product (e.g., *Citrus unshiu* fruit or the like), a residue after squeezing it and/or an enzyme-treated product of a squeezing reside or the like, to the organic solvent, the weight of the organic solvent is 10,000 times or less, preferably 1,000 times or less, further preferably 500 times or less, based on 1 weight of the material to be subjected to extraction.

After mixing of the material subjected to extraction with the organic solvent, it is preferable to carry out a treatment during the extraction in such a manner that the material subjected to extraction is uniformly dispersed in the solvent by stirring, an ultrasonic treatment or the like. It is preferable to carry out this treatment under such a condition that rotting hardly proceeds and cryptoxanthin is easily extracted without a loss. Illustratively, it is preferable that the temperature is 90°C or lower, preferably 80°C or lower, further preferably 40°C or lower. The extraction time is not particularly limited as long as cryptoxanthin is efficiently extracted, and it is illustratively from 1 minute to 10 days, preferably from 10 minutes to 3 days, and further preferably from 10 minutes to 24 hours. In addition, for the purpose of suppressing the loss of cryptoxanthin during the extraction operation, operation for reducing the dissolved oxygen, such as bubbling of nitrogen or the like or addition of an anti-oxidation substance such as vitamin E or the like may be carried out.

The amount of the dietary fibers based on cryptoxanthin is considerably reduced in the cryptoxanthin-containing compositions produced by the above-mentioned two methods, so that they are particularly preferable as the highly absorbable oral administration composition of the present invention. When such a cryptoxanthin highly absorbable oral administration composition is ingested, this composition may be optionally made into a preferable form. Illustratively, powders, solutions, tablets, granules, capsules, soft capsules, gels, pastes, syrups, suspensions, emulsions, drinks and the like may be cited. Among these, the oils and fats which are used when a solution is made by dissolving in oils and fats are not particularly limited as long as they are edible, and safflower oil, olive oil, rapeseed oil, soybean oil, corn oil, coconut oil, sunflower oil, rice oil, fish oil, beef tallow, lard, MCT and the like may be used.

Also, as the emulsifying agent which is used for preparing an emulsion, it is not particularly limited as long as they are edible, and commercially available emulsifying agents for food such as a glycerol fatty acid ester, a sucrose fatty acid ester, saponin, lecithin and the like may be used. Among them, a glycerol fatty acid ester and a sucrose fatty acid ester are preferable because they have a high effect to improve absorbability and/or absorption rate into the living body.

Further, the cryptoxanthin highly absorbable oral administration composition of the present invention may be ingested by mixing it with a food or drink, a feed, a medicine and the like. As the medicine, it can be ingested in the form of powders, tablets, granules, capsules, suspensions, syrups, solutions for internal use, troches and the like. In addition, as the food or drink of the present invention, all the foods and/or drinks such as general food, food for specified health uses, healthy food, functional food and the like are included therein. Said foods and/or drinks are not particularly limited, and for example, in addition to the above-mentioned medicine-like forms, staple foods such as bread, noodles, buckwheat noodles, boiled rice and the like, foods such as cheese, Vienna, sausage, ham, fish meat processed product and the like, confectioneries such as ice cream, cookies jellies, puddings, candies, chewing gums, yogurt, Gummy, chocolates, biscuits and the like, seasonings such as jam and the like, and drinks such as fruit juice drinks, soft drinks, liquors, nourishing drinks, tea, milk and the like may be cited.

As the feed, it can be produced by mixing the highly absorbable oral administration composition of the present invention with, for example, cereals such as corn, wheat, barley, rye and the like, brans such as wheat bran, rice bran and the like, refuses such as corn gluten meal, corn jam meal and the like, animal feeds such as skimmed milk, whey, fish meal, powdered bones and the like, yeasts such as beer yeast and the like, calciums such as calcium phosphate, calcium carbonate and the like, vitamins, oils and fats, amino acids, saccharides and the like. Regarding the use of the feed, it can be used for a pet food, a livestock feed, a feed for farming fish and the like.

### EXAMPLES

The following describes Examples of the test. In this connection, the present invention is not limited to the Examples. In this connection, in the Examples, measuring methods of cryptoxanthin and dietary fibers in the compositions, the measuring method of cryptoxanthin in serum and the measuring method of absorbability into the living body were all carried out by the methods which are the same with the above-mentioned methods.

### Example 1

1 g of a pectinase enzyme preparation for food processing use, Sumizyme PX (manufactured by SHIN-NIHON-KAGAKU-KOGYO, pectinase 5,000 units/g, arabanase 90 units/g), and 1 g of a cellulase/hemicellulase enzyme preparation, Cellulase Y-NC (manufactured by YAKULT PHARMACEUTICAL INDUSTRY Co,. Ltd., cellulase 30,000 units/g), were added to 800 g of the residue after squeezing fruit juice from *Citrus unshiu* (orange juice lees, moisture percentage: about 90%), and after thorough mixing, a static reaction was carried out at room temperature for 8 hours. This reaction liquid was centrifuged to discard the supernatant and then stirred after water was added, and the supernatant was again discarded by centrifugation. This precipitate was dried with a freeze dryer and pulverized into powder with a mixer-type pulverizer. As a result of analysis of the composition of this powder, it contained 0.5% of β-cryptoxanthin (free form-basis) and 24.6% of dietary fibers. That is, the dietary fiber amount was about 49 times the amount of cryptoxanthin.

### Comparative Example 1

The orange juice lees used in Example 1 were freeze-dried and pulverized into powder by a mixer-type pulverizer. As a result of analysis of the composition of this powder, it contained 0.02% of β-cryptoxanthin and 18.4% of dietary fibers. That is, the dietary fiber amount was 920 times the amount of cryptoxanthin.

### Test Example 1

25 mg of the powder composition produced in Example 1 was prepared into hard capsules, a healthy person was allowed to ingest one capsule for each morning and evening every day (ingested amounts per day were β-cryptoxanthin: 0.25 mg and dietary fibers: 12.3 mg), and a blood sample was collected 4 weeks thereafter to measure the β-cryptoxanthin level in the serum. As a control, 125 mg of the powder composition produced in Comparative Example 1 was prepared into hard capsules in the same manner, a healthy person was allowed to ingest five capsules thereof for each morning and evening every day (ingested amounts per day were β-cryptoxanthin: 0.25 mg and dietary fibers: 230 mg), and a blood sample was collected 4 weeks thereafter to measure the β-cryptoxanthin level in the serum.

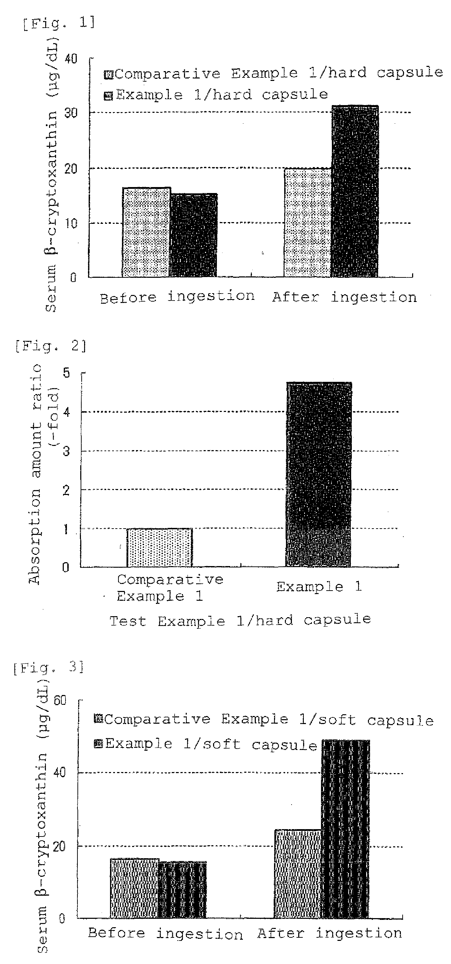

As a result, the serum β-cryptoxanthin level of the hard capsule of Example 1-ingestion group was significantly higher than that of the hard capsule of Comparative Example 1-ingestion group (Fig. 1). In addition, when increase in the serum β-cryptoxanthin level before and after the ingestion in Comparative Example 1 was regarded as 1, increase in the serum β-cryptoxanthin level before and after the ingestion in Example 1 was 4.7, so that the absorbability was improved (Fig. 2).

### Test Example 2

25 mg of the powder composition produced in Example 1 was wetted with a double amount of safflower oil and prepared into soft capsules, a healthy person was allowed to ingest one capsule thereof for each morning and evening every day, and a blood sample was collected 4 weeks thereafter to measure the β-cryptoxanthin level in the serum. As a control, 125 mg of the powder composition produced in Comparative Example 1 was wetted with a double amount of safflower oil and prepared into soft capsules in the same manner, a healthy person was allowed to ingest five capsules thereof for each morning and evening every day, and a blood sample was collected 4 weeks thereafter to measure the β-cryptoxanthin level in the serum.

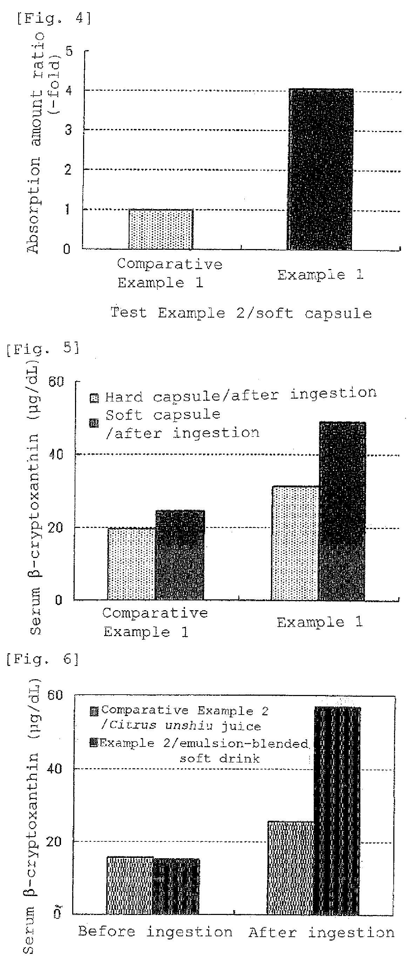

As a result, the serum β-cryptoxanthin level of the soft capsule of Example 1-ingestion group was significantly higher than that of the soft capsule of Comparative Example 1-ingestion group (Fig. 3). In addition, when increase in the serum β-cryptoxanthin level before and after the ingestion in Comparative Example 1 was regarded as 1, increase in the serum β-cryptoxanthin level before and after the ingestion in Example 1 was 4.1, so that the absorbability was improved (Fig. 4).

Comparison of Test Example 1 and Test Example 2 is shown in Fig. 5. The serum β-cryptoxanthin level of the soft capsule of Example 1-ingestion group was significantly higher than that of the hard capsule of Example 1-ingestion group. On the other hand, the serum β-cryptoxanthin level of the soft capsule of Comparative Example-1 ingestion group was higher than that of the hard capsule of Comparative Example 1-ingestion group, but significant difference was not found.

### Example 2

1L of ethanol was added to 100 g of the powder composition produced in Example 1 and, after stirring for 1 hour, an ethanol extract was obtained by filtration. After concentration by evaporating ethanol from this extract using an evaporator, a *Citrus unshiu* extract was obtained by re-dissolution in 100 ml of ethanol and subsequent filtration.

This *Citrus unshiu* extract was mixed with 100 ml of RYOTO® POLYGLYCEROL ESTER S-24-D (manufactured by Mitsubishi Chemical Corporation) and 800 ml of distilled water, followed by stirring at 45°C for 10 minutes to prepare a *Citrus unshiu* emulsion. As a result of analyzing the composition of this emulsion, 0.04% of β-cryptoxanthin (free form-basis) was contained in the emulsion. On the other hand, the dietary fibers were at the detection limit or less.

### Comparative Example 2

Edible part of *Citrus unshiu* obtained by removing the rind was squeezed using a juicer, and a *Citrus unshiu* juice was prepared by removing the solid matter using gauze. 0.0008% of β-cryptoxanthin and 0.5% of dietary fibers (Prosky method) were contained in this *Citrus unshiu* juice. That is, the dietary fibers amount was 625 times the amount of cryptoxanthin.

### Test Example 3

A soft drink was produced by thoroughly mixing the emulsion produced in Example 2 with the following raw materials and water and adjusted to 100 ml.

| | | |
|---|---|---|
| Raw materials | *Citrus unshiu* emulsion | 2 ml |
| | Dextrose syrup | 2.5 g |
| | Citric acid | 1.5 g |
| | Sodium ascorbate | 50 mg |

A healthy person was allowed to ingest 30 ml of this soft drink every morning (β-cryptoxanthin 0.24 mg-ingested amount per day), and a blood sample was collected 4 weeks thereafter to measure the β-cryptoxanthin level in the serum. As a control, a healthy person was allowed to ingest 30 ml of the *Citrus unshiu* juice produced in Comparative Example 2 every morning (β-cryptoxanthin 0.24 mg and dietary fibers 150 mg-ingested amounts per day), and a blood sample was collected 4 weeks thereafter to measure the β-cryptoxanthin level in the serum.

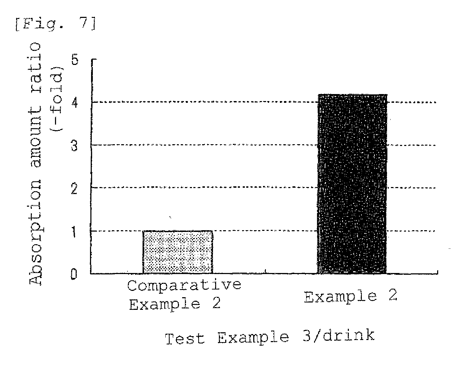

As a result, the serum β-cryptoxanthin level of the soft drink of Example 2-ingestion group was significantly higher than that of the *Citrus unshiu* juice of Comparative Example 2-ingestion group (Fig. 6). In addition, when increase in the serum β-cryptoxanthin level before and after the ingestion in Comparative Example 2 was regarded as 1, increase in the serum β-cryptoxanthin level before and after the ingestion in Example 2 was 4.2, so that the absorbability was improved (Fig. 7).

### Example 3 [Production of biscuits]

The powder composition produced in Example 1 was thoroughly mixed with the following raw materials and blending ratio and then formed and baked in an oven to produce a biscuit having orange flavor.

| | | |
|---|---|---|
| Raw materials | Powder composition | 5% |
| | Wheat flour | 50% |
| | Sugar | 20% |
| | Liquid egg | 5% |
| | Butter | 18.5% |
| | Calcium carbonate | 1% |
| | Sodium chloride | 0.5% |

### Example 4 [Production of tablets]

The powder composition produced in Example 1 was thoroughly mixed with the following raw materials and blending ratio and then subjected to tablet making to produce tablets.

| | | |
|---|---|---|
| Raw materials | Powder composition | 40% |
| | Egg shell calcium | 5% |
| | Crystalline cellulose | 10% |
| | Reduced maltose | 43% |
| | Sucrose fatty acid ester | 2% |

### Example 5 [Production of health drinks]

The *Citrus unshiu* emulsion produced in Example 2 was thoroughly mixed with the following raw materials and adjusted to 100 ml to produce health drinks.

| | | |
|---|---|---|
| Raw materials | *Citrus unshiu* emulsion | 600 mg |
| | Dextrose syrup | 500 mg |
| | Nicotinamide | 20 mg |
| | Vitamin B₁ nitrate | 5 mg |
| | Vitamin B₂ phosphate | 5 mg |
| | Vitamin B₆ | 5 mg |
| | Anhydrous caffeine | 50 mg |

### Example 6 [Production of jelly]

The *Citrus unshiu* emulsion produced in Example 2 was thoroughly mixed with the following materials and adjusted to 100 ml to produce orange-taste jelly.

| | | |
|---|---|---|
| Raw materials | *Citrus unshiu* extract | 600 mg |
| | Granulated sugar | 20 g |
| | Gelatin | 4 g |

### Example 7 [Production of ice cream]

The *Citrus unshiu* emulsion produced in Example 2 was thoroughly mixed with the following materials to produce a β-cryptoxanthin-enriched low calorie ice cream.

| | | |
|---|---|---|
| Raw materials | *Citrus unshiu* extract | 600 mg |
| | Bean curd | 80 g |
| | Milk | 30 ml |
| | Yolk | 25 g |
| | Corn starch | 20 g |
| | Reduced starch syrup | 10 g |
| | Sucralose | 40 mg |
| | Vanilla extract | 0.01 mg |

While the present invention has been described in detail and with reference to specific embodiments thereof, it will be apparent to one skilled in the art that various changes and modifications can be made therein without departing from the spirit and scope of the present invention.
This application is based on a Japanese patent application filed on July 28, 2006 (Japanese Patent Application No. 2006-206315), the entire contents thereof being thereby incorporated by reference.

### INDUSTRIAL APPLICABILITY

The present invention provides a highly absorbable oral administration composition with improved absorbability of cryptoxanthin into the living body, which can be used in foods, feeds, pharmaceutical preparations and the like. By improving absorbability of cryptoxanthin into the living body, various effects such as the provitamin A activity and anti-oxidation action, carcinogenesis inhibitory action, osteogenesis accelerating action, skin whitening action and the like possessed by cryptoxanthin can be improved.

## Claims

1. A cryptoxanthin highly absorbable oral administration composition, which is a composition comprising natural product-derived cryptoxanthin, wherein the amount of dietary fibers contained in said composition is 400 times by weight or less based on cryptoxanthin.

2. The cryptoxanthin highly absorbable oral administration composition described in claim 1, wherein absorbability of cryptoxanthin into the living body is improved 2 times or more in comparison with the case of directly ingesting a natural product containing said cryptoxanthin.

3. The cryptoxanthin highly absorbable oral administration composition described in claim 1 or 2, wherein the natural product is *Citrus unshiu*.

4. The cryptoxanthin highly absorptive oral administration composition described in any one of claims 1 to 3, further comprising an oil or fat, wherein a part or whole of cryptoxanthin is dissolved in said oil or fat.

5. The cryptoxanthin highly absorbable oral administration composition described in any one of claims 1 to 3, further comprising an emulsifying agent, wherein a part or whole of cryptoxanthin is emulsified by said emulsifying agent.

6. A method of producing the cryptoxanthin highly absorbable oral administration composition described in any one of claims 1 to 5, which comprises treating a natural product or a processed product thereof with an enzyme, subjecting the resulting enzyme-treated product to a solid-liquid separation to obtain the residue, and obtaining an oral administration composition from said residue.

7. A method of producing the cryptoxanthin highly absorbable oral administration composition described in any one of claims 1 to 5, which comprises extracting cryptoxanthin from a natural product or a processed product thereof with an organic solvent, and obtaining an oral administration composition from said extract.

8. A food or drink or a feed, which comprises the cryptoxanthin highly absorbable oral administration composition described in any one of claims 1 to 5.

9. A pharmaceutical preparation, which comprises the cryptoxanthin highly absorbable oral administration composition described in any one of claims 1 to 5, as an active ingredient.
